(19) Europäisches Patentamt
European Patent Office
Office européen des brevets

(11) **EP 2 029 221 B1**

(12) **EUROPEAN PATENT SPECIFICATION**

(45) Date of publication and mention
of the grant of the patent:
**05.08.2009 Bulletin 2009/32**

(21) Application number: **07748806.2**

(22) Date of filing: **30.05.2007**

(51) Int Cl.:
**A61N 1/16** (2006.01)

(86) International application number:
**PCT/UA2007/000034**

(87) International publication number:
**WO 2007/149060 (27.12.2007 Gazette 2007/52)**

(54) **DEVICE FOR PROTECTION AGAINST EXPOSURE TO ENERGY**

VORRICHTUNG ZUM SCHUTZ VOR ENERGIEAUSSETZUNG

DISPOSITIF DE PROTECTION CONTRE L'EXPOSITION À L'ÉNERGIE

(84) Designated Contracting States:
**AT BE BG CH CY CZ DE DK EE ES FI FR GB GR
HU IE IS IT LI LT LU LV MC MT NL PL PT RO SE
SI SK TR**
Designated Extension States:
**AL BA HR MK RS**

(30) Priority: **19.06.2006 UA 2006006881**

(43) Date of publication of application:
**04.03.2009 Bulletin 2009/10**

(73) Proprietor: **Volkov, Aleksey Yevgenyevich
Kharkov 61006 (UA)**

(72) Inventor: **Volkov, Aleksey Yevgenyevich
Kharkov 61006 (UA)**

(74) Representative: **Ripamonti, Enrico et al
Giambrocono & C. S.p.A.
Via Rosolino Pilo, 19/b
20129 Milano (IT)**

(56) References cited:
**WO-A-20/04089053          DE-U1- 9 302 948
DE-U1- 29 617 718          RU-C1- 2 259 215**

**Description**

FIELD OF THE INVENTION

The invention relates to the protection of biological objects such as humans, animals, *etc*. against exposure to electromagnetic radiations, which accompany the operation of electric and power devices, and may be practiced at home and in everyday life.

DESCRIPTION OF THE PRIOR ART

In the process of his or her everyday life, a human is continuously in a common energy and information field that is generated as a result of the addition of fields of various radiation sources, *viz.*, electromagnetic radiation, cosmic radiation, geomagnetic radiation, *etc*. An energy and information frequency spectrum of living matter affecting both physical and physiological process by means of the common energy and information field has been determined using mathematical simulation methods. To protect biological objects (for instance, humans and animals), devices were created to protect against exposure to energy. Such devices belong to the class of apparatuses used to reduce electromagnetic radiation, which accompanies the operation of electric and power devices, and to reduce the exposure of biological objects to electromagnetic radiations. The effectiveness of devices of such type has been proved experimentally.

Known in the prior art is a device for protection against exposure to energy comprising a dielectric plate with two effective surfaces and metal applicators arranged thereon. The first effective surface of the dielectric plate includes applicators in the shape of three-, four-, five-, six- and eight-beam stars arranged thereon, and the second effective surface of the dielectric plate includes two applicators arranged oppositely thereon, each of which is made in the shape of a saw-toothed polygon (Russian Patent No. 2259215, IPC A 61 N 1/16, published on August 27, 2005). This device is also provided with an applicator in the shape of a seven-beam star. This prior art device features an additional dielectric plate with applicators in shape of the spiral of Archimedes arranged thereon.

A disadvantage of the prior art device for protection against exposure to energy is that it is of a low effectiveness. This is due to that it fails to ensure a necessary polarization degree of electromagnetic radiation created by an external radiation source this resulting in an insufficient reduction in the external electromagnetic radiation and in an insufficient absorption of the external electromagnetic radiation to which a biological object is exposed. The largest portion of electromagnetic radiation harmful for humans is in a high-frequency spectrum, which spectrum is neutralized, in the prior art device, by means of one eight-beam applicator, the power of which is insufficient this having been proved by experimental investigations. Furthermore, this device is provided with metal ties magnetized in one direction. It should be pointed out, however, that these ties are demagnetized when the device is in use this decreasing the effectiveness of its functioning. It should be also mentioned that, due to the employment of the additional dielectric plate in the device and, as a consequence, increase in the spatial volume thereof, undesired energy fluxes are generated that decrease the effectiveness of functioning of the device. Another essential disadvantage of the prior art device is that the connection of applicators to each other results in increase in stray currents and, as a consequence, in the unbalance of effects of the applicators on shielding properties of the prior art device, that is to say, its properties to reduce electromagnetic radiations, which accompany the operation of electric and power devices.

The most similar to the device in accordance with this invention is a device for protection against exposure to energy comprising a dielectric plate with two effective surfaces and metal applicators arranged thereon. The first effective surface of the dielectric plate includes applicators in the shape of three-, four-, five-, six- and eight-beam stars arranged thereon, and the second effective surface of the dielectric plate includes two applicators arranged oppositely thereon, each of which is made in the shape of a saw-toothed polygon (Russian Patent No. 2259215, IPC A 61 No. 1/16, published on August 27, 2005). This device is also provided with an applicator in the shape of a seven-beam star, which is arranged on the first effective surface of the dielectric plate. This prior art device is characterized in that the second effective surface of the dielectric plate includes, in addition to two saw-toothed applicators arranged oppositely thereon, two applicators in the shape of a triangle and an eight-beam star additionally arranged thereon. Another feature of this prior art device is that the applicators arranged on the first effective surface of the dielectric plate and made in the shape of the three-; four-, six- and eight-beam stars contact to each other, and two applicators arranged on the first effective surface of the dielectric plate and made in the shape of the five- and seven-beam stars also contact to each other. On the second effective surface of the dielectric plate, all the applicators contact to each other.

A disadvantage of the prior art device for protection against exposure to energy is that it is of a low effectiveness. This is due to that it fails to ensure a necessary polarization degree of electromagnetic radiation created by an external radiation source this resulting in an insufficient reduction in the external electromagnetic radiation and in an insufficient absorption of the external electromagnetic radiation to which a biological object is exposed. The use of only six applicators arranged on the first effective surface of the dielectric plate, one of which is made in the shape of a seven-beam star, does not ensure an intensive polarization of electromagnetic radiation. The use of an eight-beam applicator along with the seven-beam applicator on the first effective surface of the dielectric plate does not ensure an effective harmonization of polarization vectors and reduces the operating frequency range of the prior art device this being a cause of a low effectiveness of its functioning. The connection of applicators to each other results in increase in stray currents and, as a consequence, in the unbalance of effects of the applicators on shielding properties of the prior art device this decreasing the effectiveness of its functioning.

The largest portion of electromagnetic radiation harmful for humans is in a high-frequency spectrum, which portion is neutralized, in the prior art device, by means of one eight-beam applicator, the power of which is insufficient this having been proved by experimental investigations. The shape and configuration of the applicators arranged on the second effective surface of the dielectric plate do not allow both uniform and intensive reduction in a high-frequency electromagnetic radiation to be achieved.

DISCLOSURE OF THE INVENTION

Accordingly, the present invention has as its object to provide a device for the protection of biological objects against exposure to energy, characterized by improved capabilities, that is to say, capabilities of reducing effectively electromagnetic radiations, which accompany the operation of electric and power devices.

The object of the invention is achieved in that, in the prior art device for protection against exposure to energy comprising a dielectric plate with two effective surfaces and metal applicators arranged thereon, wherein the first effective surface of the dielectric plate includes applicators in the shape of three-, four-, five-, six- and eight-beam stars arranged thereon, and the second effective surface of the dielectric plate includes two applicators arranged oppositely thereon, each of which is made in the shape of a saw-toothed polygon, in accordance with the invention, the first effective surface of the dielectric plate is provided with five additional applicators, one of which is made in the shape of an eight-beam star and the other four applicators are made in the shape of obtuse-angled triangles, which form a four-blade impeller and are arranged between the three-, four-, five-, and eight-beam stars, and the obtuse angle of each of the triangle is oriented towards the larger side of its adjacent triangle.

In at least one embodiment of the device for protection against exposure to energy in accordance with this invention, the dielectric plate is coated with a dielectric material layer.

In another embodiment of the device for protection against exposure to energy in accordance with this invention, the areas of the oppositely arranged applicators on the second effective surface of the dielectric plate are equal to each other and the area of each applicator is:

$$0.37 < S_1/S_2 \leq 0.43, \qquad (1)$$

where:

$S_1$ is the area of the applicator made in the shape of a saw-toothed polygon, in $mm^2$; and

$S_2$ is the area of the second effective surface of the dielectric plate, in $mm^2$, and the minimum distance (h) between said two applicators is:

$$L/44 < h < L/21, \qquad (2)$$

where:

h is the minimum distance between the two applicators made in the shape of saw-toothed polygons, in mm; and

L is the width of the dielectric plate, in mm.

In yet another embodiment of the device for protection against exposure to energy in accordance with this invention, the total area of all of the applicators arranged on the first effective surface of the dielectric plate is:

$$0.38 < S_3/S_2 \leq 0.77, \qquad (3)$$

where:

S_3 is the total area of all of the applicators arranged on the first effective surface of the dielectric plate, in $mm^2$; and

S_2 is the area of the second effective surface of the dielectric plate, in $mm^2$.

In yet another embodiment of the device for protection against exposure to energy in accordance with this invention, each of the two saw-toothed applicators comprises an equal number of beams; preferably, the number of beams of each saw-toothed applicator is not less than seven and not more than twenty-one.

In yet another embodiment of the device for protection against exposure to energy in accordance with this invention, the four applicators made in the shape of obtuse-angled triangles have their obtuse angle of between 108° and 122°.

This invention provides reduction in (increase in the level of dumping) an electromagnetic radiation, which accompanies the operation of electric and power devices, due to increase in polarization degree thereof with the aid of the device in accordance with the invention. This becomes possible thanks to providing the device in accordance with the invention with five additional applicators arranged on the first effective surface of the dielectric plate. The orientation chosen of the beams of the obtuse-angled triangles, which form a four-blade impeller, results in producing a local source of an elevated strength of a field induced in the device in accordance with the invention, the vector whereof is directed opposite to the vector of the external electromagnetic radiation field this increasing the polarization degree of the space around the device and, as a consequence, resulting in a significant reduction in the external high-frequency electromagnetic radiation. The placement of the four-blade impeller between three-, four-, five-, and eight-beam applicators enables the device in accordance with the invention to function within a wide frequency range of the external electromagnetic radiation. Thanks to the use of the additional applicator in the shape of an eight-beam star, the effectiveness of functioning of the device within a high-frequency range of the radiation spectrum is improved greatly.

Coating the dielectric plate with the applicators arranged thereon with a dielectric material layer prevents the applicators from being contacted to atmospheric air and their microstructure from being damaged in order to preserve predetermined characteristics of the applicators. Making the applicators in compliance with (1), (2) and 3) and choosing the obtuse angle of each of the four additional applicators made in the shape of obtuse-angled triangles of between 108° and 122° make it possible to attain the uniformity of damping a high-frequency electromagnetic radiation.

It was found, as result of investigations, that the device in accordance with this invention reduces effectively the exposure of biological objects, in particular, humans, to electromagnetic radiations.

BRIEF DESCRIPTION OF THE DRAWINGS

Fig. 1 is a general view a device for protection against exposure to energy in accordance with this invention.

Fig. 2 is a view of a first effective surface of a dielectric plate.

Fig. 3 is a view of a second effective surface of the dielectric plate.

DESCRIPTION OF THE PREFERRED EMBODIMENT

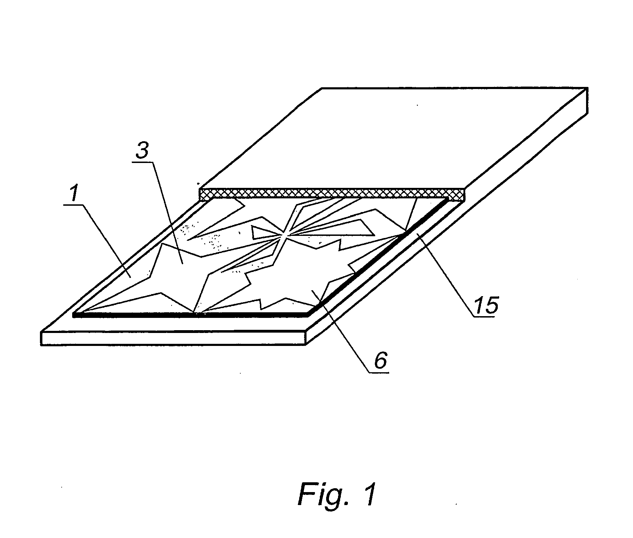

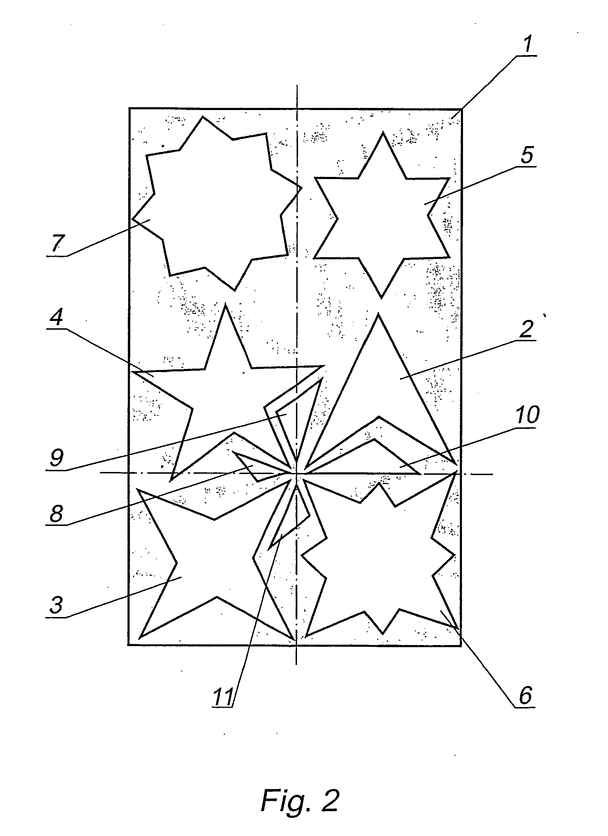

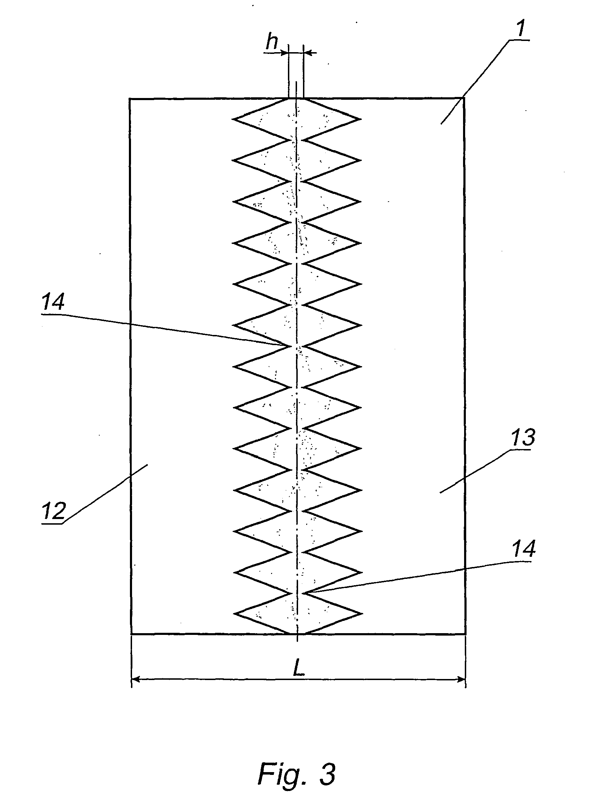

Referring first to Fig. 1, a device for protection against exposure to energy comprises a dielectric plate 1 that includes a first effective surface and a second effective surface located on both sides of the dielectric plate 1. The first effective surface of the dielectric plate 1 includes metal applicators arranged thereon (see Fig. 2): an applicator 2 made in the shape of a three-beam star; an applicator 3 made in the shape of a four-beam star; an applicator 4 made in the shape of a five-beam star; an applicator 5 made in the shape of a six-beam star; and an applicator 6 made in the shape of an eight-beam star. Also, arranged additionally on the first effective surface of the dielectric plate 1 are: an applicator 7 made in the shape of a eight-beam star and four additional applicators 8, 9, 10, 11 made in the shape of obtuse-angled triangles which form a four-blade impeller and are arranged between the applicators 3, 4, 2, 6, respectively. The obtuse angle of each of the applicators 3, 4, 2, 6 is oriented towards the larger side of its adjacent triangle and is between 108° and 122°. The second effective surface of the dielectric plate 1 (see Fig. 3) includes two applicators 12 and 13 arranged oppositely thereon. The two applicators 12 and 13 are made in the shape of saw-toothed polygons. Each of the applicators 12, 13 comprises an equal number of beams 14, *viz.,* fourteen beams, as can be seen in Fig. 3.

The area $S_1$ of each applicator 12, 13 is equals to $0.391S_2$, where $S_2$ is the area of the second effective surface of the dielectric plate 1. The minimum distance (h) between these applicators is 0.034L.

The total area $S_3$ of all of the applicators 2 to 11 arranged on the first effective surface of the dielectric plate 1 is within the range predetermined by (3).

Referring again to Fig. 1, the dielectric plate 1 is coated with a dielectric material layer 15 which protects the dielectric plate 1 against damages when in use.

The device for protection against exposure to energy in accordance with this invention functions as follows:

The device is placed between an electromagnetic radiation source and a biological object (for instance, a human) to be protected. The applicators 2 to 13 convert this electromagnetic radiation so that external field radiation parameters in the active area of the device approach zero. This conversion occurs thanks to the superposition of the vectors of the external field and those of a field induced in the device. Both magnitude and direction of the induced field vector are added to the external field vector this resulting in a significant reduction in the external field and, as a consequence, in an essential decrease in the exposure of the biological object to it. The applicators 8 to 11 that form the four-blade impeller amplify the field induced in the device and provide the polarization of the space around the device this resulting in an effective reduction in the external electromagnetic radiation.

The placement of the applicators 8 to 11 between the applicators 3, 4, 2, 6 widens the operation range of the device. The arrangement of two saw-toothed applicators 12 and 13, each of which comprises fourteen beams 14, on the second effective surface of the dielectric plate 1 creates uniformity and ensures an intensive damping of a high-frequency electromagnetic radiation.

**Claims**

1. A device for protection against exposure to energy comprising a dielectric plate (1) with two effective surfaces and metal applicators arranged thereon, wherein the first effective surface of the dielectric plate includes metal applicators in the shape of three-, four-, five-, six- and eight-beam stars arranged thereon, and the second effective surface of the dielectric plate includes two metal applicators arranged oppositely thereon, each of which is made in the shape of a saw-toothed polygon, **characterized in that** the first effective surface of the dielectric plate is provided with five additional metal applicators, one of which is made in the shape of an eight-beam star (7) and the other four applicators are made in the shape of obtuse-angled triangles (8, 9, 10, 11), which form a four-blade impeller and are arranged between the three-, four-, five-, and eight-beam stars, and the obtuse angle of each of the triangle is oriented towards the larger side of its adjacent triangle.

2. The device for protection against exposure to energy of Claim 1, **characterized in that** the dielectric plate is coated with a dielectric material layer.

3. The device for protection against exposure to energy of Claim 1, **characterized in that** the areas of the oppositely arranged applicators on the second effective surface of the dielectric plate are equal to each other and the area of each applicator is:

$$0.37 < S_1/S_2 \leq 0.43$$

where:

$S_1$ is the area of the applicator made in the shape of a saw-toothed polygon, in $mm^2$; and
$S_2$ is the area of the second effective surface of the dielectric plate, in $mm^2$, and the minimum distance (h) between said two applicators is:

$$L/44 < h < L/21$$

where:

h is the minimum distance between the two applicators made in the shape of saw-toothed polygons, in mm; and
L is the width of the dielectric plate, in mm.

4. The device for protection against exposure to energy of Claim 1, **characterized in that** the total area of all of the applicators arranged on the first effective surface of the dielectric plate is:

$$0{,}38 < S_3/S_2 \leq 0{,}77$$

where:

> $S_3$ is the total area of all of the applicators arranged on the first effective surface of the dielectric plate, in mm$^2$; and
> $S_2$ is the area of the second effective surface of the dielectric plate, in mm$^2$.

5. The device for protection against exposure to energy of Claim 1, **characterized in that** each of the two saw-toothed applicators comprises an equal number of beams; preferably, the number of beams of each saw-toothed applicator is not less than seven and not more than twenty-one.

6. The device for protection against exposure to energy of Claim 1, **characterized in that** the four applicators made in the shape of obtuse-angled triangles have their obtuse angle of between 108° and 122°.


**Patentansprüche**

1. Vorrichtung zum Schutz vor Energieaussetzung, umfassend eine dielektrische Platte (1) mit zwei wirksamen Oberflächen und darauf angeordnete Applikatoren, wobei die erste wirksame Oberfläche der dielektrischen Platte darauf angeordnete Metallapplikatoren in der Form von drei-, vier-, fünf-, sechs-und acht-strahligen Sternen umfasst und die zweite wirksame Oberfläche der dielektrischen Platte zwei Metallapplikatoren umfasst, die darauf gegenüberliegend angeordnet sind, von denen jeder in der Form eines Sägezahn-Polygons hergestellt ist, **dadurch gekennzeichnet, dass** die erste wirksame Oberfläche der dielektrischen Platte mit fünf zusätzlichen Metallapplikatoren versehen ist, von denen einer in der Form eines acht-strahligen Sterns (7) hergestellt ist und die anderen vier Applikatoren in der Form von stumpfwinkligen Dreiecken (8, 9, 10, 11) hergestellt sind, die ein vierblättriges Flügelrad bilden und zwischen den drei-, vier-, fünf- und acht-strahligen Sternen angeordnet sind, und wobei der stumpfe Winkel jedes Dreiecks in Richtung der größeren Seite seines benachbarten Dreiecks orientiert ist.

2. Vorrichtung zum Schutz vor Energieaussetzung gemäß Anspruch 1, **dadurch gekennzeichnet, dass** die dielektrische Platte mit einer dielektrischen Materialschicht beschichtet ist.

3. Vorrichtung zum Schutz vor Energieaussetzung gemäß Anspruch 1, **dadurch gekennzeichnet, dass** die Flächen der gegenüberliegend angeordneten Applikatoren auf der zweiten wirksamen Oberfläche der dielektrischen Platte einander gleich sind und die Fläche jedes Applikators ist:

$$0{,}37 < S_1/S_2 \leq 0{,}43$$

worin:

> $S_1$ die Fläche des Applikators, hergestellt in Form eines Sägezahn-Polygons, in mm$^2$ ist und
> $S_2$ die Fläche der zweiten wirksamen Oberfläche der dielektrischen Platte in mm$^2$ ist, und der Mindestabstand (h) zwischen den zwei Applikatoren ist:

$$L/44 < h < L/21$$

worin:

> h der Mindestabstand zwischen den zwei Applikatoren, hergestellt in Form von Sägezahn-Polygonen, in mm ist und
> L die Breite der dielektrischen Platte in mm ist.

**4.** Vorrichtung zum Schutz vor Energieaussetzung gemäß Anspruch 1, **dadurch gekennzeichnet, dass** die gesamte Fläche aller Applikatoren, die auf der ersten wirksamen Oberfläche der dielektrischen Platte angeordnet sind, ist:

$$0,38 < S_3/S_2 \leq 0,77$$

worin:

S3 die gesamte Fläche aller Applikatoren, die auf der ersten wirksamen Oberfläche der dielektrischen Platte angeordnet sind, in mm$^2$ ist und
S2 die Fläche der zweiten wirksamen Oberfläche der dielektrischen Platte in mm$^2$ ist.

**5.** Vorrichtung zum Schutz vor Energieaussetzung gemäß Anspruch 1, **dadurch gekennzeichnet, dass** jeder der zwei Sägezahn-Applikatoren eine gleiche Anzahl von Strahlen umfasst, wobei die Anzahl der Strahlen jedes Sägezahn-Applikators vorzugsweise nicht weniger als 7 und nicht mehr als 21 ist.

**6.** Vorrichtung zum Schutz vor Energieaussetzung gemäß Anspruch 1, **dadurch gekennzeichnet, dass** die vier Applikatoren, die in der Form von stumpfwinkligen Dreiecken hergestellt sind, ihren stumpfen Winkel mit zwischen 180° und 122° haben.

## Revendications

**1.** Dispositif de protection contre l'exposition à l'énergie comprenant une plaque diélectrique (1) avec deux surfaces efficaces et des applicateurs métalliques agencés dessus, dans lequel la première surface efficace de la plaque diélectrique comprend des applicateurs métalliques en forme d'étoiles à trois, quatre, cinq, six et huit branches agencés dessus, et la seconde surface efficace de la plaque diélectrique comprend deux applicateurs métalliques agencés en face à face dessus, chacun ayant la forme d'un polygone en dents de scie, **caractérisé en ce que** la première surface efficace de la plaque diélectrique est munie de cinq applicateurs métalliques supplémentaires, dont l'un a la forme d'une étoile à huit branches (7), et les quatre autres applicateurs ont la forme de triangles obtusangles (8, 9, 10, 11), qui forment une roue à quatre aubes et sont agencés entre les étoiles à trois, quatre, cinq et huit branches, et l'angle obtus de chaque triangle est orienté vers le côté le plus grand de son triangle adjacent.

**2.** Dispositif de protection contre l'exposition à l'énergie selon la revendication 1, **caractérisé en ce que** la plaque diélectrique est revêtue d'une couche de matériau diélectrique.

**3.** Dispositif de protection contre l'exposition à l'énergie selon la revendication 1, **caractérisé en ce que** les aires des applicateurs agencés en face à face sur la seconde surface efficace de la plaque diélectrique sont égales et l'aire de chaque applicateur est :

$$0,37 < S_1/S_2 \leq 0,43$$

où :

$S_1$ est l' aire de l' applicateur en forme de polygone en dents de scie en mm$^2$ ; et
$S_2$ est l'aire de la seconde surface efficace de la plaque diélectrique, en mm$^2$, et la distance minimale (h) entre lesdits deux applicateurs est :

$$L/44 < h < L/21$$

où :

h est la distance minimale entre les deux applicateurs en forme de polygone en dents de scie, en mm ; et

L est la largeur de la plaque diélectrique, en mm.

4. Dispositif de protection contre l'exposition à l'énergie selon la revendication 1, **caractérisé en ce que** l'aire totale de tous les applicateurs agencés sur la première surface efficace de la plaque diélectrique est :

$$0,38 < S_3/S_2 \leq 0,77$$

où :

$S_3$ est l'aire totale de tous les applicateurs agencés sur la première surface efficace de la plaque diélectrique, en mm$^2$ ; et
$S_2$ est l'aire de la seconde surface efficace de la plaque diélectrique, en mm$^2$.

5. Dispositif de protection contre l'exposition à l'énergie selon la revendication 1, **caractérisé en ce que** chacun des deux applicateurs en dents de scie comprend un nombre égal de branches ; de préférence, le nombre de branches de chaque applicateur en dents de scie n'est pas inférieur à sept et n'est pas supérieur à vingt et un.

6. Dispositif de protection contre l'exposition à l'énergie selon la revendication 1, **caractérisé en ce que** l'angle obtus des quatre applicateurs en forme de triangle obtusangle est compris entre 108° et 122°.

Fig. 1

*Fig. 2*

Fig. 3

**REFERENCES CITED IN THE DESCRIPTION**

**Patent documents cited in the description**

- RU 2259215